# EUROPEAN PATENT SPECIFICATION

(11) **EP 3 165 201 B1**
(45) Date of publication and mention of the grant of the patent: **24.04.2019**
(21) Application number: 15814880.9
(22) Date of filing: 22.06.2015
(51) Int. Cl.: A61F 7/03

(54) **DISPOSABLE BODY WARMER**
EINWEG-KÖRPERWÄRMER
ELÉMENT DE RÉCHAUFFEMENT CORPOREL JETABLE

(30) Priority: 02.07.2014 JP 2014137183
(43) Date of publication of application: 10.05.2017
(73) Proprietor: DAINIHON JOCHUGIKU CO., LTD., Osaka-shi Osaka 550-0001 (JP)
(72) Inventor: GEHO, Hirofumi, Toyonaka-shi Osaka 561-0827 (JP); TAMURA, Chiaki, Toyonaka-shi Osaka 561-0827 (JP); ASAI, Hiroshi, Toyonaka-shi Osaka 561-0827 (JP); NAKAYAMA, Koji, Toyonaka-shi Osaka 561-0827 (JP)
(74) Representative: Intès, Didier Gérard André
(86) International application number: PCT/JP2015/067852
(87) International publication number: WO 2016/002555

(56) References cited:
- EP-A1- 0 989 834
- WO-A1-2014/021269
- JP-A- 2009 062 250
- JP-A- 2013 042 963
- JP-A- 2013 176 549
- JP-A- 2013 176 549

## Description

### TECHNICAL FIELD

The present invention relates to disposable warmers which have a fragrance-releasing effect.

### BACKGROUND ART

Disposable warmers are used by many people as an everyday commodity which can easily make or keep the user warm. Various types of disposable warmers are available on the market, such as those which are inserted in clothes in use, those which are attached to the back or foot, those which are inserted in shoes, those which are worn around the neck like mufflers, and the like. User requirements have in recent years diversified, and the above various types of disposable warmers have been required to have a fragrance-releasing capability. However, disposable warmers have a peculiar smell which is caused by their raw materials, and such a smell is found unpleasant in use by a significant number of users. Disposable warmers are mainly made of an iron powder, an activated carbon, water, a salt, a water retention agent, and the like. In order to impart a fragrance-releasing capability to a disposable warmer, a fragrance material is added to the above raw materials. At this time, the activated carbon, which is one of the raw materials, adsorbs the fragrance material, which poses a problem that it is difficult for the fragrance material to exhibit the fragrance-releasing effect.

To address this problem, a heat generating tool (disposable warmer) has been previously proposed which includes a characteristic blend of raw materials to increase the fragrance-releasing capability (see, for example, Patent Document 1). Patent Document 1 indicates that the use of an exothermic composition including an oxidation accelerating agent having an iodine adsorption performance of 500 mg/g or less, an oxidizable metal powder, and water, as a raw material for a disposable warmer, allows a fragrance material included in the disposable warmer to exhibit the fragrance-releasing effect without being significantly adsorbed by an activated carbon in use.

Also, a heat generating tool (disposable warmer) has been propsed in which an oxidation accelerating agent having a lower iodine adsorption performance is used (see, for example, Patent Document 2). Patent Document 2 indicates that an oxidation accelerating agent which is selected as a raw material for a disposable warmer has an iodine adsorption performance of 400 mg/g, which is lower than that of Patent Document 1, and a sequestering agent is further used in combination. Patent Document 2 also indicates that the use of such raw materials can prevent the disappearance of or changes in the fragrance-releasing capability of a disposable warmer while inhibiting an unpleasant smell derived from the exothermic composition.

### CITATION LIST

### PATENT LITERATURE

Patent Document 1: Japanese Unexamined Patent Application Publication No. 2013-176549
Patent Document 2: Japanese Unexamined Patent Application Publication No. 2014-30486

JP2013176549 discloses a disposable warmer comprising an exothermic composition and a fragrance material.

### SUMMARY OF INVENTION

### TECHNICAL PROBLEM

In the disposable warmers of Patent Documents 1 and 2, an oxidation accelerating agent having a low iodine adsorption performance is used in order to inhibit a fragrance material from being adsorbed by the oxidation accelerating agent so that the desired fragrance-releasing effect is exhibited. However, the use of an oxidation accelerating agent having a low iodine adsorption performance leads to a decrease in the amount of oxygen taken into the warmer, so that the oxidation reaction of the iron powder does not smoothly proceed, which poses another problem that heat cannot be stably generated. In addition, when the iodine adsorption performance of the oxidation accelerating agent is low, some iron powder remains unreacted with oxygen later in the use of the disposable warmer. In such a case, the unpleasant smell derived from the iron powder become strong. Therefore, even when the fragrance-releasing effect of the fragrance material is exhibited, the user feels the unpleasant smell derived from the iron powder more strongly than the fragrance of the fragrance material.

Thus, there has been room for an improvement in the achievement of the fragrance-releasing capability and the exothermicity simultaneously in conventional disposable warmers. With the above problems in mind, the present invention has been made. It is an object of the present invention to provide a disposable warmer which can exhibit the fragrance-releasing effect of the fragrance material and keep generating heat stably while reducing an unpleasant smell derived from the iron powder.

### SOLUTION TO PROBLEM

In order to achieve the above object, a disposable warmer according to the present invention and claim 1 includes:
an air-permeable bag;
an exothermic composition including an iron powder, an activated carbon, a salt, a water absorbent resin, and water;
a fragrance material; and
a fragrance material adsorption inhibition agent for inhibiting adsorption of the fragrance material into the activated carbon,
   wherein
the exothermic composition, the fragrance material, and the fragrance material adsorption inhibition agent are enclosed in the bag.

As described above, one of the purposes of adding the fragrance material to the exothermic composition including an iron powder, an activated carbon, a salt, a water absorbent resin, and water, which are raw materials for the disposable warmer, is to reduce an unpleasant smell derived from the iron powder. However, the above problem arises when the fragrance material is simply added to the exothermic composition, which makes it difficult to achieve sufficient performance of the disposable warmer. Under these circumstances, the present inventors have extensively studied to find that the above problem can be eliminated or reduced by using a "fragrance material adsorption inhibition agent" for inhibiting the adsorption of the fragrance material into the activated carbon. The fragrance material adsorption inhibition agent has the effect of inhibiting the adsorption of the fragrance material into the activated carbon. Therefore, when a fragrance material is added to the disposable warmer, the fragrance material is allowed to exhibit a fragrance-releasing effect. In addition, the fragrance material adsorption inhibition agent does not significantly inhibit the oxygen adsorption effect of the activated carbon, and therefore, oxygen in air is successfully taken into the disposable warmer, so that an exothermic oxidation reaction between the iron powder and oxygen proceeds smoothly, and heat can be stably generated from early in the use of the disposable warmer. In addition, later in the use of the disposable warmer, substantially all the iron powder has been oxidized and consumed, so that substantially no unpleasant smell derived from the iron powder occurs, and even if any such a smell occurs, the smell is subtle.

Thus, in the disposable warmer having this configuration, heat can be stably generated from early to later in use while the fragrance material is allowed to exhibit the fragrance-releasing effect. In addition, the disposable warmer of the present invention can be expected to provide a relaxing effect of the fragrance while warming the user's body.

In the disposable warmer of the present invention,
a fragrance material which is the same as or different from the fragrance material is preferably attached to or included in the bag.

Although, in the above disposable warmer, the exothermic composition, the fragrance material, and the fragrance material adsorption inhibition agent are enclosed in the air-permeable bag, the fragrance material may be attached or included to or in the bag in advance. With such a bag, the fragrance material included as a raw material and the fragrance material attached to or included in the bag exhibit their fragrance-releasing effects synergistically. Therefore, the user can actually feel the fragrance of the fragrance material more strongly, and does not substantially feel an unpleasant smell derived from the iron powder. In addition to the thermal sensation causing effect of the disposable warmer, the above fragrance-releasing effect can provide a high relaxing effect to the user.

To achieve the above object, a disposable warmer according to the present invention and claim 3 includes:
an air-permeable bag to or in which a fragrance material is attached or included;
an exothermic composition including an iron powder, an activated carbon, a salt, a water absorbent resin, and water; and
a fragrance material adsorption inhibition agent for inhibiting the adsorption of the fragrance material into the activated carbon,
   wherein
the exothermic composition and the fragrance material adsorption inhibition agent are enclosed in the bag.

In the disposable warmer having this configuration, a fragrance material is attached to or included in the air-permeable bag containing raw materials for the warmer, such as an iron powder and the like, in order to reduce an unpleasant smell derived from the iron powder, and allow the fragrance material to exhibit a fragrance-releasing effect. Here, the use of the "fragrance material adsorption inhibition agent" as a raw material inhibits the adsorption of the fragrance material into the activated carbon, so that the fragrance material is allowed to exhibit the fragrance-releasing effect. In addition, the fragrance material adsorption inhibition agent does not significantly inhibit the oxygen adsorption effect of the activated carbon, and therefore, oxygen in air is successfully taken into the disposable warmer, so that an exothermic oxidation reaction between the iron powder and oxygen proceeds smoothly, and heat can be stably generated from early in the use of the disposable warmer. In addition, later in the use of the disposable warmer, substantially all the iron powder has been oxidized and consumed, so that substantially no unpleasant smell derived from the iron powder occurs, and even if any such a smell occurs, the smell is subtle.

Note that, in the disposable warmer having this configuration, the fragrance material is attached to or included in the bag instead of being included in the raw materials, and therefore, the fragrance material is less likely to come into direct contact with the activated carbon, and as a result, the adsorption of the fragrance material into the activated carbon is further inhibited.

In the disposable warmer of the present invention,
a fragrance material which is the same as or different from the fragrance material is preferably additionally enclosed in the bag.

Although, in the above disposable warmer, the fragrance material is attached to or included in the bag, an fragrance material may be further enclosed in the bag (i.e., a fragrance material may be included in the raw materials for the disposable warmer). With such a disposable warmer, the fragrance material attached to or included in the bag and the fragrance material included as a raw material exhibit their fragrance-releasing effects synergistically. Therefore, the user can actually feel the fragrance of the fragrance material more strongly, and does not substantially feel an unpleasant smell derived from the iron powder. In addition to the thermal sensation causing effect of the disposable warmer, the above fragrance-releasing effect can provide a high relaxing effect to the user.

In the disposable warmer of the present invention,
the fragrance material adsorption inhibition agent preferably includes a low active substance having an iodine adsorption performance lower than the iodine adsorption performance of the activated carbon.

The disposable warmer having this configuration employs a fragrance material adsorption inhibition agent including a low active substance having an iodine adsorption performance lower than that of the activated carbon, and therefore, the fragrance material adsorption performance of the activated carbon can be reduced. As a result, the fragrance material is sufficiently diffused from the disposable warmer in use, and therefore, is allowed to exhibit the fragrance-releasing effect.

In the disposable warmer of the present invention,
the low active substance is preferably a precursor of the activated carbon before activation.

In the disposable warmer having this configuration, the use of an activated carbon precursor before activation as the low active substance, can successfully reduce the fragrance material adsorption performance of the activated carbon. In addition, the activated carbon precursor is a raw material for the activated carbon which is an oxidation accelerating agent for the disposable warmer, and therefore, has excellent affinity when mixed in the exothermic composition. Therefore, the activated carbon precursor is not likely to cause a deterioration in performance, an alteration in quality, or the like of the disposable warmer, and has excellent keeping qualities.

In the disposable warmer of the present invention,
the low active substance is preferably a carbonaceous material.

In the disposable warmer having this configuration, the use of a carbonaceous material as the low active substance can successfully reduce the fragrance material adsorption performance of the activated carbon. In addition, carbonaceous materials are similar to the activated carbon which is an oxidation accelerating agent for the disposable warmer in terms of ingredients, and therefore, have excellent affinity when mixed with the exothermic composition. Therefore, carbonaceous materials are not likely to cause a deterioration in performance, an alteration in quality, or the like of the disposable warmer, and have excellent keeping qualities.

In the disposable warmer of the present invention,
the average value of the iodine adsorption performance of the activated carbon and the iodine adsorption performance of the fragrance material adsorption inhibition agent is adjusted to 510-700 mg/g.

In the disposable warmer having this configuration, the average value of the iodine adsorption performance of the activated carbon and the iodine adsorption performance of the fragrance material adsorption inhibition agent is adjusted to 510-700 mg/g. Therefore, the effect of inhibiting the activated carbon from adsorbing the fragrance material, and the exothermic oxidation reaction between oxygen adsorbed by the activated carbon and the iron powder, can be excellently balanced. As a result, the fragrance material can be allowed to exhibit the fragrance-releasing effect, and heat generation can be kept stable, while an unpleasant smell derived from the iron powder can be reduced.

In the disposable warmer of the present invention,
the mixture ratio of the activated carbon and the fragrance material adsorption inhibition agent is adjusted to 1.4:1 to 4:1.

In the disposable warmer having this configuration, the mixture ratio of the activated carbon and the fragrance material adsorption inhibition agent is adjusted to 1.4:1 to 4:1. Therefore, the effect of inhibiting the activated carbon from adsorbing the fragrance material, and the exothermic oxidation reaction between oxygen adsorbed by the activated carbon and the iron powder, can be excellently balanced. As a result, the fragrance material can be allowed to exhibit the fragrance-releasing effect, and heat generation can be kept stable, while an unpleasant smell derived from the iron powder can be reduced.

In the disposable warmer of the present invention,
the activated carbon is preferably an oxidation reaction acceleration agent.

In the disposable warmer having this configuration, the activated carbon which is an oxidation reaction acceleration agent accelerates the exothermic oxidation reaction between oxygen and the iron powder, and therefore, the performance of the disposable warmer can be enhanced. The present invention is disclosed in independent claims 1 and 3; preferred embodiments are disclosed in the dependent claims.

### BRIEF DESCRIPTION OF DRAWINGS

FIG. 1 is a model diagram for describing behaviors of an activated carbon, fragrance material, and fragrance material adsorption inhibition agent in a disposable warmer according to a first embodiment of the present invention.
FIG. 2 is a model diagram for describing behaviors of an activated carbon, fragrance material, and fragrance material adsorption inhibition agent in a disposable warmer according to a second embodiment of the present invention.

### DESCRIPTION OF EMBODIMENTS

Embodiments relating to a disposable warmer according to the present invention will now be described. Note that the present invention is not intended to be limited to configurations described in the embodiments below and shown in the accompanying drawings.

### <Exothermic Composition>

A disposable warmer according to the present invention includes, as a main component, an exothermic composition including an iron powder, an activated carbon, a salt, a water absorbent resin, and water. By adjusting the ingredients of the exothermic composition, the time it takes for the warmer to start generating heat, the highest temperature during heat generation, and the duration for which the warmer keeps generating heat, and the like, can be controlled. The ingredients of the exothermic composition will now be described in greater detail.

The iron powder is a metal powder which reacts with oxygen in air to cause an exothermic oxidation reaction. Examples of the iron powder which is a raw material for the warmer include reduced iron powder, cast iron powder, atomized iron powder, electrolytic iron powder, and the like. These may be used alone or in combination. The iron powder may also be a mixture of granular powder, fibrous powder, and the like.

The iron powder may be mixed with other metal powders. Examples of such metal powders include zinc powder, aluminum powder, magnesium powder, copper powder, nickel powder, tin powder, and the like. These metal powders may be used alone or in combination. The metal powder may also be a mixture of granular powder, fibrous powder, and the like.

The activated carbon has a large specific surface which provides excellent adsorption performance, and therefore, functions as an oxidation reaction acceleration agent which efficiently takes oxygen in air into the disposable warmer, and accelerates an exothermic oxidation reaction between oxygen and the iron powder. As a result, the performance (duration and temperature) of the disposable warmer can be enhanced. The activated carbon is classified according to its shape. The activated carbon used in the disposable warmer is mainly powdery activated carbon or granular activated carbon. Examples of a raw material for the powdery activated carbon include sawdust, hard woodchips, charcoal, peat, and the like. Examples of a raw material for the granular activated carbon include charcoal, coconut husk charcoal, coal (lignite, brown coal, bituminous coal, anthracite, etc.), oil carbon, phenol resin, and the like. These raw materials are activated by a treatment such as, for example, activation by gas, activation by a chemical agent, or the like. The granular activated carbon may be further divided into crushed or ground carbon, powdered carbon, granulated carbon, and bead carbon. Note that, in addition to the above activated carbons, any materials that have excellent oxygen adsorption performance and can accelerate an exothermic oxidation reaction between oxygen and the iron powder (oxidation reaction acceleration agent), can be used as a raw material for the disposable warmer.

The salt has the function of accelerating the oxidation of the iron powder. Examples of the salt include sodium chloride, calcium chloride, magnesium chloride, aluminum chloride, potassium chloride, zinc chloride, and the like. These salts may be used alone or in combination.

Here, although water is included together with the salt in the raw materials in order to accelerate the oxidation of the iron powder, the addition of water to the raw materials causes the iron powder to be sticky. With this in mind, a water absorbent resin is further included in the raw materials in order to prevent the iron powder from becoming sticky and keep the iron powder dry or smooth. Examples of the water absorbent resin include an isobutylene-maleic anhydride copolymer, a polyvinyl alcohol-acrylic acid copolymer, a starch-acrylic acid salt graft copolymer, a polyacrylic acid salt cross-linked product, an acrylic acid salt-acrylic acid ester copolymer, an acrylic acid salt-acrylamide copolymer, a hydrolysate of a polyacrylonitrile cross-linked product, a polyaspartic acid salt-based resin, a polyglutamic acid salt-based resin, a polyalginic acid salt-based resin, and the like. These water absorbent resins may be used alone or in combination.

A moisture retention agent may be used for the same purpose of the water absorbent resin. Examples of the moisture retention agent include vermiculite, pearlite, calcium silicate, kaolinite, talc, smectite, mica, bentonite, calcium carbonate, silica gel, alumina, zeolite, silicon dioxide, wood flour, cotton, and the like. These water retention agents may be used alone or in combination. In addition, the moisture retention agent may be used in combination with the water absorbent resin. Moreover, two or more moisture retention agents may be used in combination with the water absorbent resin.

The water has the function of accelerating the oxidation of the iron powder in cooperation with the salt. Examples of the water include distilled water, tap water, ion-exchanged water, industrial water, and the like.

A disposable warmer according to the present invention may include, in addition to the exothermic composition, a hydrogen generation inhibitor, such as a weak acid salt of an alkali metal, a weak acid salt of an alkaline-earth metal, a metasilicate, or the like, a thickener, an excipient, a surfactant, or the like. The contents of these ingredients may be adjusted as appropriate according to the ratio of the ingredients included in the exothermic composition, the amount of the exothermic composition contained in a bag, the purpose of use of the disposable warmer, or the like.

### <Fragrance material>

A disposable warmer according to the present invention includes a fragrance material in order to exhibit a fragrance-releasing effect or a relaxing effect in addition to a thermal sensation causing effect. Examples of the fragrance material include: monoterpene ketones such as galaxolide, musk ketone, hexyl cinnamic aldehyde, ethylene brassylate, methyl atrarate, hexyl salicylate, oranger crystal, terpineol, citronellol, geraniol, nerol, linalool, dihydrolinalool, tetrahydrolinalool, dihydromyrcenol, menthol, borneol, isoborneol, citronellal, citral, dimethyl octanal, carvone, dihydrocarvone, pulegone, menthone, tricyclodecenyl acetate, ambroxide, cashmeran, calone, heliotropin, indolarome, indole, methyl cedryl ketone, methyl β-naphthyl ketone, methyldihydrojasmonate, rosephenone, 7-acetyl-1,2,3,4,5,6,7,8-octahydro-1,1,6,7-tetramethyl naphthalene, acetoacetic acid-m-xylidide, acetoacetic acid-o-toluidide, acetosyringone, acetyl triethyl citrate, benzophenone, benzyl benzoate, benzyl caprylate, benzyl cinnamate, benzyl eugenol, benzyl laurate, benzyl methyl tiglate, benzyl phenyl ether, benzyl phenyl acetate, benzyl salicylate, geranyl anthranilate, geranyl hexanoate, geranyl cyclopentanone, geranyl phenyl acetate, hexyl phenyl acetate, icosane, indane, cinnamyl butyrate, cinnamyl phenyl acetate, hexenyl benzoate, citral diethyl acetal, ionone, isoamyl benzoate, linalyl octanoate, 1-menthyl salicylate, citronellyl anthranilate, dimethyl phenethyl carbonyl isobutyrate , diphenyl oxide, dodecyl butyrate, ethyl vanillate, ethyl vanillin, menthyl isovalerate, methoxy ethyl phenyl glycidate, methyl 2,4-dihydroxy-3,6-dimethyl benzoate, nerolidyl acetate, neryl isovalerate, octenyl cyclopentanone, octyl caprylate, phenethyl isoamyl ether, phenethyl octanoate, phenethyl phenyl acetate, ethyl vanillin acetate, ethyl vanillin propylene glycol acetal, ethyl hexyl palmitate, eugenyl benzoate, farnesol, farnesyl acetate, farnesyl methyl ether, formaldehyde cyclododecyl methyl acetal, formyl ethyl tetramethyl tetralin, furfuryl benzoate, γ-dodecalactone, phenethyl salicylate, phenoxy ethyl propionate, phenyl benzoate, phenyl disulfide, santalyl butyrate, tetrahydro-pseudoionone, theobromine, valencene, camphor, damascene, and the like, monoterpene aldehydes such as neral, perillaldehyde, and the like, ester compounds such as cinnamyl formate, geranyl formate, and the like, phenyl ethyl alcohol, acetophenone, allyl caproate, amyl cinnamic aldehyde, amyl salicylate, benzaldehyde, benzyl acetate, benzyl alcohol, camphor, cinnamic alcohol, coumarin, dihydrolinalool, dihydromyrcenol, diphenyl oxide, ethyl-2-methyl butyrate, hedione, hexanol, cis-3-hexanol, isoamyl acetate, lilial, methyl benzoate, methyl ionone, methyl salicylate, α-pinene, β-pinene, rose oxide, terpineol, γ-nonalactone, γ-undecalactone, vanillin, and the like. Also, floral fragrance materials, citrus fragrance materials, and various essential oils may be used, including, for example, citronella oil, orange oil, lemon oil, lime oil, yuzu oil, lavender oil, peppermint oil, cinnamon oil, eucalyptus oil, lemon eucalyptus oil, hiba cypress oil, grapefruit oil, cedar wood oil, geranium oil, white thyme oil, peppermint oil, jasmine oil, hinoki cypress oil, green tea essential oil, neroli oil, bergamot oil, petitgrain oil, lemongrass oil, vanilla oil, chamomile oil, rosemary oil, sage oil, ginger oil, ylang-ylang oil, mint oil, rose oil, lily oil, lilac oil, cardamom oil, lemongrass oil, neroli oil, sandalwood oil, anise oil, caraway oil, ambergris oil, civet oil, castoreum oil, clove oil, Japanese mugwort oil, vetiver oil, and the like. Moreover, animal-based fragrance materials obtained from animals, such as musk and the like, can be used.

The above fragrance materials may be used alone or in combination. The fragrance materials not only have a fragrance-releasing effect, but also exhibit a relaxing effect. Therefore, if a most suitable fragrance material is selected and the fragrance thereof is adjusted according to the application, usage, and the like of the disposable warmer, the disposable warmer can exhibit various types of fragrance. Here, it is preferable that a fragrance material used in a disposable warmer according to the present invention should diffuse a fragrance from early in heat generation performed by the disposable warmer, and keep a fragrance-releasing effect as long as the disposable warmer continues to be warm. Preferable examples of an fragrance material which can effectively exhibit a fragrance-releasing effect and keep the fragrance-releasing effect when the disposable warmer has a temperature of 40-60°C which is a temperature suitable for users, include Japanese mugwort oil, ginger oil, floral fragrance materials, and citrus fragrance materials.

### <Fragrance material Adsorption Inhibition Agent>

A disposable warmer according to the present invention contains, in addition to the above exothermic composition including an iron powder, an activated carbon, a salt, a water absorbent resin, and water, and the above fragrance material, a "fragrance material adsorption inhibition agent" which inhibits the activated carbon from adsorbing the fragrance material. The fragrance material adsorption inhibition agent has the function of inhibiting the activated carbon from adsorbing the fragrance material. If the fragrance material adsorption inhibition agent has been added to the raw material for the disposable warmer, when an fragrance material is further added to the disposable warmer, the fragrance material is less likely to be adsorbed by the activated carbon, and the fragrance material which has not been adsorbed by the activated carbon is diffused into the outside of the disposable warmer, so that a fragrance-releasing effect can be exhibited. In addition, the fragrance material adsorption inhibition agent does not significantly inhibit the oxygen adsorption effect of the activated carbon, and therefore, oxygen in air is successfully taken into the disposable warmer, so that an exothermic oxidation reaction between the iron powder and oxygen proceeds smoothly, and heat can be stably generated from early in the use of the disposable warmer. Later in the use of the disposable warmer, substantially all the iron powder has been oxidized and consumed, so that substantially no unpleasant smell derived from the iron powder occurs, and even if any such a smell occurs, the smell is subtle.

The above fragrance material adsorption inhibition agent preferably includes a low active substance which has an iodine adsorption performance lower than that of the activated carbon. An example of such a low active substance is a precursor of the activated carbon (activated carbon precursor before activation). Here, the production of the activated carbon will be briefly described. As the activated carbon of the disposable warmer, powdery activated carbon or granular activated carbon is used as described above. Powdery activated carbon is produced by impregnating the raw material such as charcoal or the like with a chemical such as zinc chloride, phosphoric acid, or the like, or carbonizing the raw material, followed by activation, purification, and pulverization. Granular activated carbon is produced by performing pulverization/molding, carbonization, activation, and purification/sieving on the raw material such as charcoal or the like. Granular activated carbon is produced mainly by three different methods having different orders in which the above steps are performed. In a first production method, the raw material is subjected to pulverization/molding, carbonization, activation, and purification/sieving, in that order. In a second production method, the raw material is subjected to carbonization, pulverization, activation, and purification/sieving, in that order. In a third production method, the raw material is subjected to carbonization, pulverization/molding, activation, and purification/sieving, in that order.

Therefore, when the final product is powdery activated carbon, the raw material before activation, i.e., a precursor of the activated carbon (activated carbon precursor before activation), can be used as the low active substance having an iodine adsorption performance lower than that of the activated carbon. As such an activated carbon precursor before activation, the raw material which has been impregnated with a chemical such as zinc chloride, phosphoric acid, or the like, the raw material which has been carbonized, can be used. Also, when the final product is granular activated carbon, the raw material which has been subjected to pulverization/molding, the raw material which has been subjected to pulverization/molding and then carbonization, or the like can be used as in the case of powdery activated carbon. When such a low active substance having an iodine adsorption performance lower than that of the activated carbon is included in the fragrance material adsorption inhibition agent, the fragrance material adsorption performance of the activated carbon can be reduced, and the fragrance material is sufficiently diffused into the outside of the disposable warmer in use, and therefore, a fragrance-releasing effect can be exhibited.

When the fragrance material adsorption inhibition agent is an activated carbon precursor before activation, the fragrance material adsorption performance of the activated carbon can be successfully reduced. As a result, the fragrance-releasing effect of the fragrance material can be more strongly exhibited. Note that the activated carbon precursor is a raw material for the activated carbon which is an oxidation accelerating agent for the disposable warmer, and therefore, has excellent affinity when mixed in the exothermic composition. Therefore, the activated carbon precursor is not likely to cause a deterioration in performance, an alteration in quality, or the like of the disposable warmer, and has excellent keeping qualities.

Moreover, carbonaceous materials may be used as the above low active substance. As used herein, the term "carbonaceous material" means any raw material for activated carbon in a broad sense, or an activated carbon precursor before activation in a narrow sense. Examples of carbonaceous materials include charcoal, bamboo charcoal, coal, and the like, which are a raw material for activated carbon, i.e., a carbonized material before activation. The use of a carbonaceous material as the low active substance can successfully reduce the fragrance material adsorption performance of activated carbon. In addition, carbonaceous materials are similar to activated carbons which are an oxidation accelerating agent for the disposable warmer in terms of ingredients, and therefore, have excellent affinity when mixed with the exothermic composition. Therefore, carbonaceous materials are not likely to cause a deterioration in performance, an alteration in quality, or the like of the disposable warmer, and have excellent keeping qualities.

The iodine adsorption performance is adjusted so that the average value of the iodine adsorption performance of the activated carbon and the iodine adsorption performance of the fragrance material adsorption inhibition agent is 510-700 mg/g, more preferably 530-690 mg/g, and even more preferably 550-650 mg/g. Here, the "average value of iodine adsorption performances" is a value obtained by directly measuring a mixture of the activated carbon and the fragrance material adsorption inhibition agent (actually measured value). Alternatively, the "average value of the iodine adsorption performances" may be a weighted average of the iodine adsorption performance of the activated carbon and the iodine adsorption performance of the fragrance material adsorption inhibition agent (calculated value). When the average value of the iodine adsorption performances is less than 510 mg/g, the amount of oxygen which reacts with the iron powder is small, and therefore, heat is less likely to be stably generated. When the average value of the iodine adsorption performances is more than 700 mg/g, the function of the fragrance material adsorption inhibition agent decreases, and therefore, it is difficult to achieve a fragrance-releasing effect. When the average value of the iodine adsorption performances is adjusted within the above range, the effect of inhibiting the activated carbon from adsorbing the fragrance material, and the exothermic oxidation reaction between oxygen adsorbed by the activated carbon and the iron powder, can be excellently balanced. As a result, the fragrance material can be allowed to exhibit the fragrance-releasing effect, and heat generation can be kept stable, while an unpleasant smell derived from the iron powder can be reduced.

### <Bag>

The exothermic composition, fragrance material, and fragrance material adsorption inhibition agent which are raw materials for the disposable warmer of the present invention are enclosed in a bag having air permeability. As such a bag, any bag can be used that has air permeability, does not leak the raw materials such as the exothermic composition and the like, is resistant to heat generated by the warmer, and has such a strength that it is not easily broken. Bags used for conventional disposable warmers may be used.

The bag is formed of any material that has air permeability, preferably nonwoven fabric. A material for fibers constituting the nonwoven fabric is not particularly limited. Examples of the fiber include polyethylene terephthalate, polyethylene, polypropylene, polyamide, polylactic acid, rayon, and the like. These fibers are used alone or in combination (composite fiber). Moreover, a laminate product in which nonwoven fabric and paper are joined on top of each other, or a blended fabric such as polypropylene/rayon or the like may be used. The weight per unit area of the bag is adjusted so that the bag does not leak out the exothermic composition, and can efficiently take oxygen in air so that the exothermic oxidation reaction occurs. The weight per unit area of the bag is preferably 60-100 g/m².

A bag used in the disposable warmer of the present invention may be obtained by attaching a porous film and a nonwoven fabric together. Examples of the porous film include polyethylene, polypropylene, polyurethanes, plastics obtained by modifying these to be softer, and the like which have minute open pores in the surface and inside. When a bag obtained by attaching a porous film and a nonwoven fabric together is used, the uneven distribution of the raw materials enclosed in the disposable warmer can be reduced. The air permeability of the bag is not particularly limited. In order to allow the disposable warmer to stably generate heat and cause the user to feel that it tightly sticks to the user's body, the air permeability of the bag is preferably about 8,000-30,000 sec/100 mL.

The bag may have a large number of pinholes so that oxygen in air is easily taken into the disposable warmer. When such pinholes are provided, oxygen in air is more efficiently taken into the disposable warmer, so that the exothermic oxidation reaction between oxygen and the iron powder is accelerated. The pinholes are formed and concentrated in a band-shaped region having a width of 3-5 cm, for example. The diameter and arrangement of the pinholes are adjusted so that the air permeability is 2-10 sec/300 cc as measured using a Gurley air permeability measuring instrument, for example.

The nonwoven fabric may be attached by various adhesion techniques, such as using an adhesive or the like. Heat fusion using a heat sealer is preferable.

The bag may include a plurality of compartments. The size, the amounts of the exothermic composition, fragrance material, and fragrance material adsorption inhibition agent, and the like, of each compartment, are adjusted as appropriate according to the usage or the like of the disposable warmer. In addition, adjacent compartments may be linked together by a linking portion along which they can be cut off each other. In order to easily separate the compartments, perforations may be provided along a center line of the linking portion. With such a configuration, the uneven distribution of the contents (the exothermic composition, the fragrance material, and the fragrance material adsorption inhibition agent) of the bag can be inhibited, so that the bag can flexibly fit on a part which bends such as the elbow, knee, and the like. In addition, by cutting along the linking portion(s) as appropriate, the bag can be changed into a desired size or shape in use.

Alternatively, an air-permeable bag to or in which the fragrance material is attached or included may be used to enclose the exothermic composition and the fragrance material adsorption inhibition agent. With such a bag, the fragrance material is less likely to come into direct contact with the activated carbon, so that the adsorption of the fragrance material into the activated carbon is further inhibited.

Moreover, the fragrance material may be included in the raw materials for the disposable warmer, and the resultant raw materials may be enclosed in an air-permeable bag to or in which the fragrance material is attached or included. In such a disposable warmer, the fragrance material included as a raw material and the fragrance material attached to or included in the bag exhibit their fragrance-releasing effects synergistically. Therefore, the user can actually feel the fragrance of the fragrance material more strongly, and does not substantially feel an unpleasant smell derived from the iron powder. In addition to the thermal sensation causing effect of the disposable warmer, the above fragrance-releasing effect can provide a high relaxing effect to the user.

A disposable warmer according to the present invention may be provided with an adhesive layer on at least a portion of the bag so that the disposable warmer can be attached to the body through a cloth or the like (adhesive type). In this case, an upper side of the adhesive layer is covered by a protective release liner, and the release liner is removed immediately before use, and the adhesive layer is attached to the body, a cloth worn on the body, or the like. The adhesive layer may have a pattern, such as a polka-dot pattern, striped pattern, or the like.

The exothermic composition, fragrance material, and fragrance material adsorption inhibition agent enclosed in the above bag are typically contained in an air-impermeable external bag. When the external bag is opened immediately before use, the disposable warmer comes into contact with oxygen, so that an exothermic oxidation reaction starts. The disposable warmer of the present invention provides sufficient warmth from early in use, and can exhibit the fragrance-releasing effect and relaxing effect of the fragrance material, and the thermal sensation causing effect, during heat generation performed by the disposable warmer.

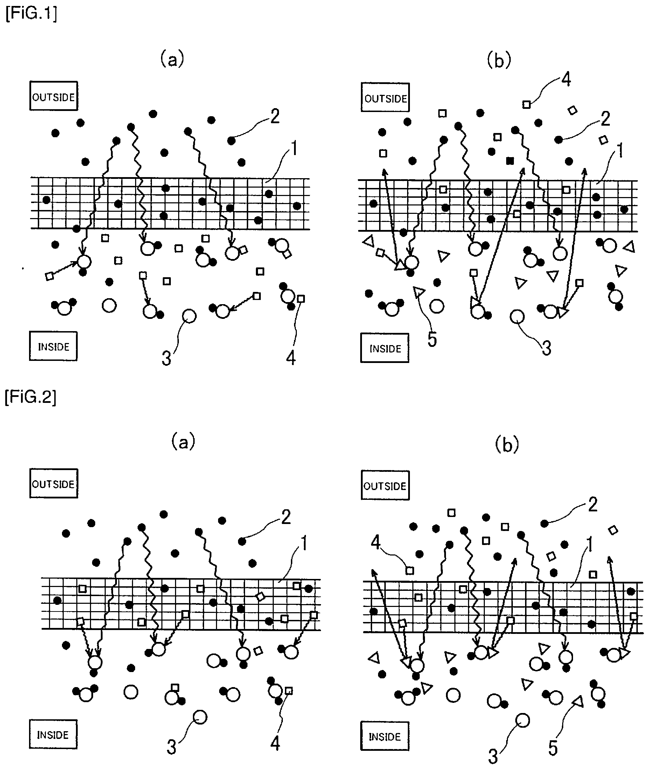

Next, two representative embodiments of the disposable warmer of the present invention will be described with reference to FIGS. 1 and 2.

### (First Embodiment)

A disposable warmer according to a first embodiment includes an air-permeable bag, and an exothermic composition including an iron powder, an activated carbon, a salt, a water absorbent resin, and water, a fragrance material, and a fragrance material adsorption inhibition agent for inhibiting the adsorption of the fragrance material into the activated carbon, which are enclosed in the bag. FIG. 1 is a model diagram for describing behaviors of the activated carbon, fragrance material, and fragrance material adsorption inhibition agent in the disposable warmer according to the first embodiment. FIG. 1(a) is a model diagram showing a conventional disposable warmer which does not contain a fragrance material adsorption inhibition agent. FIG. 1(b) is a model diagram showing a disposable warmer according to the present invention which includes a fragrance material adsorption inhibition agent as a raw material. In FIGS. 1(a) and 1(b), it is assumed that a side below the bag 1 of the disposable warmer is the inside of the disposable warmer (i.e., the raw materials are contained), and a side above the bag 1 is the outside of the disposable warmer (i.e., the atmosphere exists). In FIGS. 1(a) and 1(b), closed dots indicate oxygen 2 in air, open circles indicate an activated carbon 3, and open squares indicate a fragrance material 4. In FIG. 1(b), open triangles indicate an fragrance material adsorption inhibition agent 5. Note that the iron powder, salt, water absorbent resin, and water which are ingredients of the exothermic composition are not shown for the sake of convenience.

As shown in FIG. 1(a), the oxygen 2 in air passes through the air-permeable bag 1 and is then taken into the disposable warmer and adsorbed by the activated carbon 3. In addition, the fragrance material 4 which is enclosed together with the activated carbon 3 in the disposable warmer is gradually adsorbed by the activated carbon 3. As a result, the fragrance material 4 which can be released from the disposable warmer decreases. Therefore, in the conventional disposable warmer, it is difficult for the fragrance material 4 which is added to exhibit the fragrance-releasing effect. In contrast to this, when, as shown in FIG. 1(b), the fragrance material adsorption inhibition agent 5 is included in the raw materials, the fragrance material adsorption inhibition agent 5 inhibits the adsorption of the fragrance material 4 into the activated carbon 3, so that the fragrance material 4 is diffused into the outside of the bag 1, and therefore, the fragrance-releasing effect is exhibited. Meanwhile, the fragrance material adsorption inhibition agent 5 does not significantly inhibit the adsorption effect of the activated carbon 3 with respect to the oxygen 2, and therefore, the oxygen 2 in air is successfully taken into the disposable warmer, so that an exothermic oxidation reaction between the iron powder and the oxygen 2 proceeds smoothly, and heat can be stably generated from early in the use of the disposable warmer. In addition, later in the use of the disposable warmer, substantially all the iron powder has been oxidized and consumed, so that substantially no unpleasant smell derived from the iron powder occurs, and even if any such a smell occurs, the smell is subtle.

### (Second Embodiment)

A disposable warmer according to a second embodiment includes an air-permeable bag to or in which a fragrance material is attached or included, and an exothermic composition including an iron powder, an activated carbon, a salt, a water absorbent resin, and water, and a fragrance material adsorption inhibition agent which inhibits the adsorption of the fragrance material into the activated carbon, which are enclosed in the bag. FIG. 2 is a model diagram for describing behaviors of the activated carbon, fragrance material, and fragrance material adsorption inhibition agent in the disposable warmer according to the second embodiment. FIG. 2(a) is a model diagram showing a conventional disposable warmer which does not include a fragrance material adsorption inhibition agent. FIG. 2(b) is a model diagram showing a disposable warmer according to the present invention which includes a fragrance material adsorption inhibition agent as a raw material. In FIGS. 2(a) and 2(b), the positional relationship (inside and outside) of the disposable warmer, and the materials indicated by reference signs, are similar to those of FIGS. 1(a) and 1(b). Also, in FIGS. 2(a) and 2(b), the iron powder, salt, water absorbent resin, and water are not shown for the sake of convenience.

As shown in FIG. 2(a), the oxygen 2 in air passes through the air-permeable bag 1 and is then taken into the disposable warmer and adsorbed by the activated carbon 3. In addition, the fragrance material 4 which is attached to or included in the bag 1 of the disposable warmer is gradually adsorbed by the activated carbon 3. As a result, the fragrance material 4 attached to or included in the bag 1 decreases. Therefore, in the conventional disposable warmer, it is difficult for the fragrance material 4 attached to or included in the bag 1 to exhibit the fragrance-releasing effect. In contrast to this, when, as shown in FIG. 2(b), the fragrance material adsorption inhibition agent 5 is included in the raw materials, the fragrance material adsorption inhibition agent 5 inhibits the adsorption of the fragrance material 4 into the activated carbon 3, so that the fragrance material 4 is diffused into the outside of the bag 1, and therefore, the fragrance-releasing effect is exhibited by the fragrance material 4. Meanwhile, the fragrance material adsorption inhibition agent 5 does not significantly inhibit the adsorption effect of the activated carbon 3 with respect to the oxygen 2, and therefore, the oxygen 2 in air is successfully taken into the disposable warmer, so that an exothermic oxidation reaction between the iron powder and the oxygen 2 proceeds smoothly, and heat can be stably generated from early in the use of the disposable warmer. In addition, later in the use of the disposable warmer, substantially all the iron powder has been oxidized and consumed, so that substantially no unpleasant smell derived from the iron powder occurs, and even if any such a smell occurs, the smell is subtle.

Moreover, in the disposable warmer of the second embodiment, the fragrance material 4 is attached to or included in the bag instead of being included in the raw materials, and therefore, the fragrance material 4 is less likely to come into direct contact with the activated carbon 3, so that the adsorption of the fragrance material 4 into the activated carbon 3 is further inhibited. Therefore, the fragrance material 4 can more efficiently exhibit the fragrance-releasing effect, and substantially no unpleasant smell derived from the iron powder occurs, and even if any such a smell occurs, the smell is subtle.

### Examples

In order to verify (A) the iodine adsorption performance, (B) the fragrance-releasing effect, and (C) the temperature performance (thermal sensation causing effect) of the disposable warmer of the first embodiment, a plurality of disposable warmers (Examples 1-7) having characteristic features of the present invention were prepared and tested in terms of (A)-(C). Also, for comparison, disposable warmers (Comparative Examples 1-8) which do not have a characteristic feature of the present invention were prepared and tested in the same way.

Raw materials (ingredients) for the disposable warmers of Examples 1-7 are shown in Table 1. Of these raw materials, an iron powder, a salt, a water absorbent resin, an activated carbon, and water are included in an exothermic composition. The exothermic composition further includes a fragrance material and a fragrance material adsorption inhibition agent. In each example, a steam-activated carbon "Shirasagi PHC-14," manufactured by Japan Enviro Chemicals, Ltd., was used as the activated carbon, sodium chloride was used as the salt, and a polyacrylic acid salt cross-linked product was used as the water absorbent resin. In addition, Japanese mugwort oil (Examples 1-3 and 7), ginger oil (Example 4), a floral fragrance material (Example 5), or a citrus fragrance material (Example 6) was used as the fragrance material, and a carbonaceous material was used as the fragrance material adsorption inhibition agent. In addition, the different examples had different mixture ratios of the prepared activated carbon and carbonaceous material. Note that "others" shown in Table 1 indicate a moisture retention agent, a hydrogen generation inhibitor, and the like.

The raw materials for the disposable warmer of each example were prepared according to the mixture ratio shown in Table 1. Thereafter, the raw materials were enclosed in a bag of nonwoven fabric having an air permeability of 20,000 sec/100 mL. Thus, the disposable warmers of Examples 1-7 were produced. Meanwhile, for Comparative Examples 1-8, the same raw materials as those of the above examples are prepared for the disposable warmer of each comparative example (note that, in each comparative example, the mixture ratio of the activated carbon and the carbonaceous material is different), according to the mixture ratios shown in Table 2. Thereafter, the raw materials were enclosed in a bag similar to that of as in the above examples. Thus, the disposable warmers of Comparative Examples 1-8 were produced.

### (A) Measurement of iodine adsorption performance

The iodine adsorption performance of each disposable warmer was evaluated using a method compliant with JIS K1474. Specifically, the following steps (1)-(6) were carried out.
(1) A sample was prepared in an amount which allows the residual concentration range after iodine adsorption to include 2.5 g/L, and was pulverized until it became homogeneous.
(2) The pulverized sample was put into a 100-mL brown flask with a ground glass stopper, 50 mL of 0.05-mol/L iodine solution was put into the flask, and the flask was tightly sealed.
(3) The flask was shaken using an agitator at 210 times/min for about 15 min at room temperature so that the sample was allowed to adsorb iodine.
(4) The solid content of the sample after shaking was separated using a centrifuge.
(5) Ten milliliters of the supernatant of the sample after centrifugation was weighed, and titrated using 0.1-mol/L sodium thiosulfate solution.
(6) When the iodine yellow became thin, 1 mL of 1-wt% starch solution was added, and the titration was continued. When the blue color disappeared, the titration was ended.

The amount of iodine that is adsorbed per gram of the sample (mg/g) was calculated on the basis of the titer obtained by the above operation.

### (B) Fragrance-releasing effect

Ten subjects were asked whether or not they could feel the fragrace of the fragrance material diffused from each disposable warmer. The fragrance-releasing effect was evaluated according to the following levels.
Open circle: there were eight or more subjects who felt the fragrance
Open triangle: there were four to seven subjects who felt the fragrance
Cross: there were three or less subjects who felt the fragrance

### (C) Temperature performance (thermal sensation causing effect)

The temperature performance of each disposable warmer was evaluated using a method compliant with JIS S4100 according to the following levels.
Open circle: the highest temperature was 45-60°C, and heat was generated at a temperature of 40°C or more for a duration of 5 hours or more
Open triangle: the highest temperature was more than 40°C and less than 45°C, and heat was generated at a temperature of 40°C or more for a duration of less than 5 hours
Cross: the highest temperature was 40°C or less

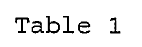

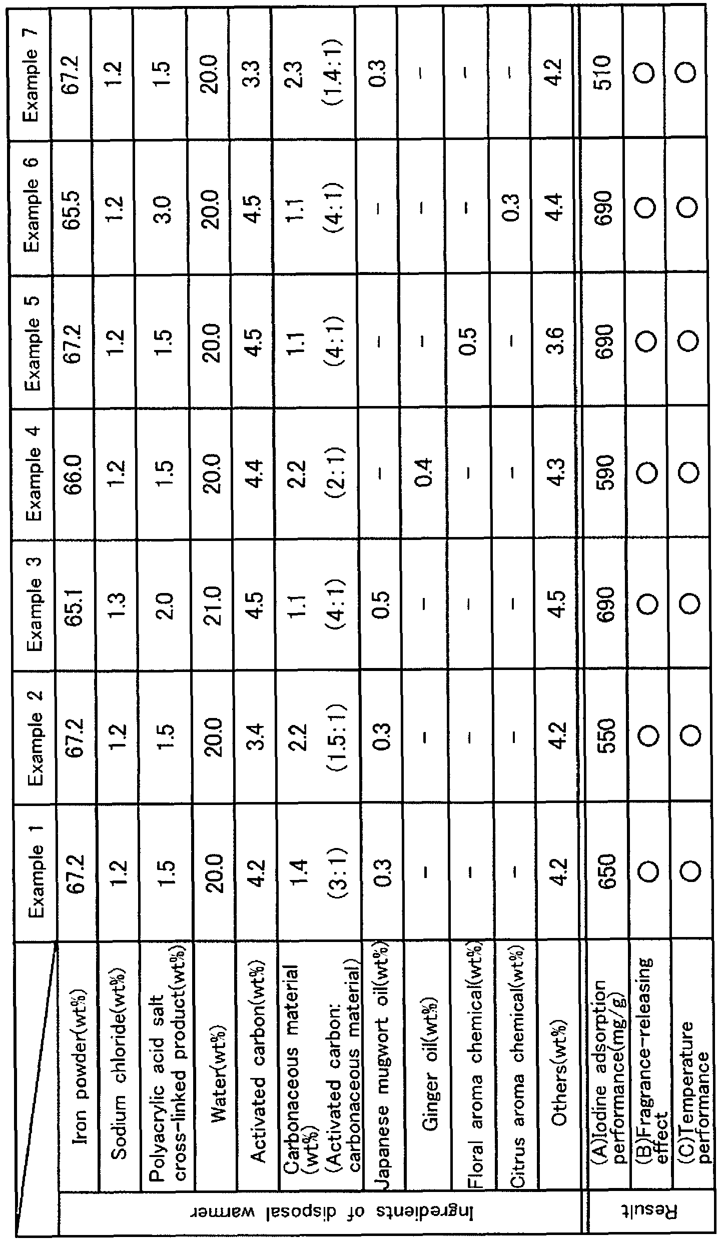

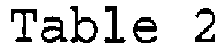

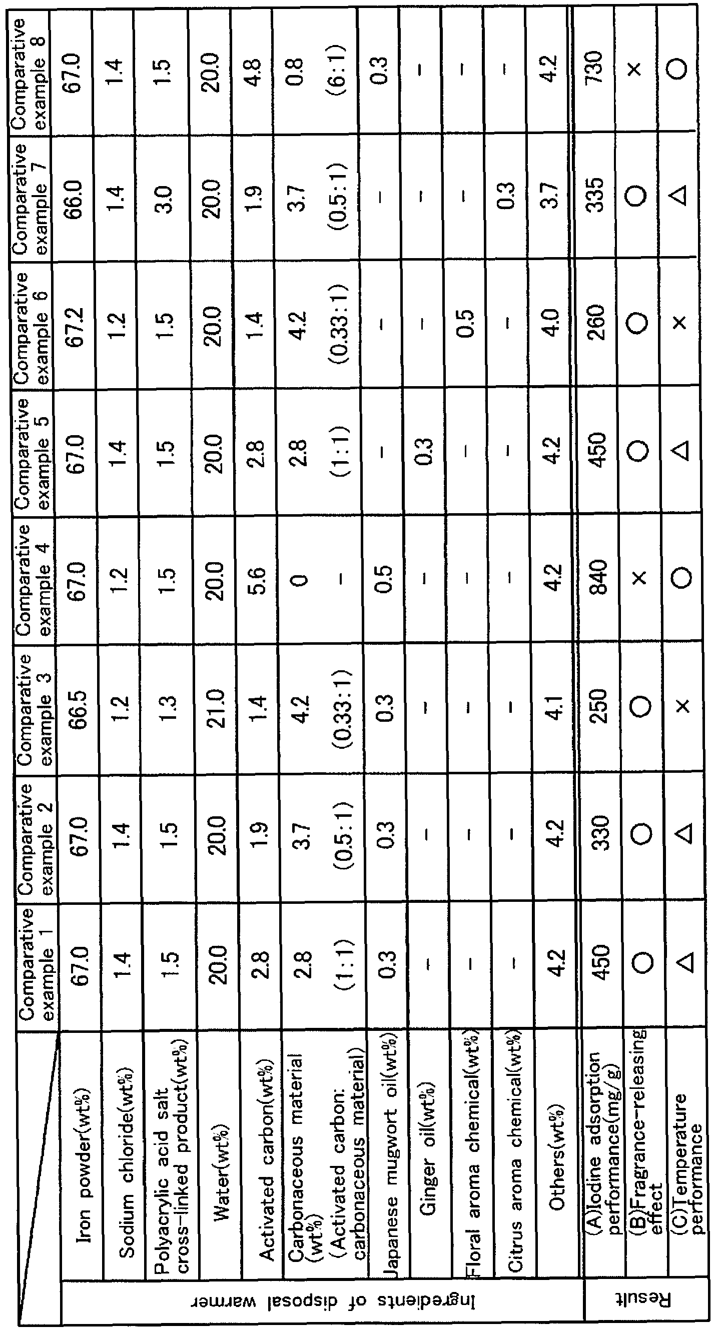

As shown in Table 1, the disposable warmers of Examples 1-7 have an iodine adsorption performance which is within an appropriate range, and therefore, simultaneously exhibited both an excellent fragrance-releasing effect and temperature performance. In contrast to this, as shown in Table 2, the disposable warmers of Comparative Examples 1-8 failed to simultaneously exhibit both an excellent fragrance-releasing effect and temperature performance. The disposable warmers of Comparative Examples 1-3 and 5-7 exhibited a good fragrance-releasing effect, and an iodine adsorption performance which was lower than that of the product of the present invention, and did not sufficiently generate heat, i.e., lower temperature performance. In particular, the disposable warmers of Comparative Examples 3 and 6 exhibited an iodine adsorption performance of as low as 250-260 mg/g, and temperature performance which was at the level indicated by the "cross." The disposable warmers of Comparative Examples 4 and 8 exhibited a high iodine adsorption performance, temperature performance which was at the high level indicated by the "open circle," and a fragrance-releasing effect which was at the level indicated by the "cross." This may be because the iodine adsorption performance is excessively high, and therefore, the fragrance material is excessively adsorbed by the activated carbon, so that the fragrance-releasing effect of the fragrance material is not exhibited.

As described above, in the disposable warmer of the present invention, the effect of inhibiting the adsorption of the fragrance material into the activated carbon, and the exothermic oxidation reaction between oxygen adsorbed by the activated carbon and the iron powder, are excellently balanced. As a result, the fragrance-releasing effect of the fragrance material can be exhibited and heat generation can be kept stable while an unpleasant smell derived from the iron powder is reduced.

### INDUSTRIAL APPLICABILITY

The disposable warmer of the present invention is applicable to both domestic and industrial fields.

### REFERENCE SIGNS LIST

- 1: BAG OF DISPOSABLE WARMER
- 2: OXYGEN
- 3: ACTIVATED CARBON
- 4: FRAGRANCE MATERIAL
- 5: FRAGRANCE MATERIAL ADSORPTION INHIBITION AGENT

## Claims

1. A disposable warmer comprising:
an air-permeable bag (1);
an exothermic composition including an iron powder, an activated carbon (3), a salt, a water absorbent resin, and water;
a fragrance material (4); and
a fragrance material adsorption inhibition agent (5) for inhibiting adsorption of the fragrance material (4) into the activated carbon (3), and
the exothermic composition, the fragrance material (4), and the fragrance material adsorption inhibition agent (5) being enclosed in the air-permeable bag (1),
wherein
the average value of the iodine adsorption performance of the activated carbon (3) and the iodine adsorption performance of the fragrance material adsorption inhibition agent (5) is adjusted to 510-700 mg/g,
**characterized in that**
the mixture ratio of the activated carbon (3) and the fragrance material adsorption inhibition agent (5) is adjusted to 1.4:1 to 4:1.

2. The disposable warmer of claim 1, **characterized in that**
a fragrance material (4) which is the same as or different from the fragrance material (4) is attached to or included in the air-permeable bag (1).

3. A disposable warmer comprising:
an air-permeable bag (1) to which a fragrance material (4) is attached;
an exothermic composition including an iron powder, an activated carbon (3), a salt, a water absorbent resin, and water; and
a fragrance material adsorption inhibition agent (5) for inhibiting the adsorption of the fragrance material (4) into the activated carbon (3), and
the exothermic composition and the fragrance material adsorption inhibition agent (5) being enclosed in the air-permeable bag (1) to which the fragrance material (4) is attached,
wherein
the average value of the iodine adsorption performance of the activated carbon (3) and the iodine adsorption performance of the fragrance material adsorption inhibition agent (5) is adjusted to 510-700 mg/g,
**characterized in that**
the mixture ratio of the activated carbon (3) and the fragrance material adsorption inhibition agent (5) is adjusted to 1.4:1 to 4:1.

4. The disposable warmer of claim 3, **characterized in that**
a fragrance material (4) which is the same as or different from the fragrance material (4) is additionally enclosed in the bag.

5. The disposable warmer of any one of claims 1-4, **characterized in that**
the fragrance material adsorption inhibition agent (5) includes a low active substance having an iodine adsorption performance lower than the iodine adsorption performance of the activated carbon (3).

6. The disposable warmer of claim 5, **characterized in that**
the low active substance is a precursor of the activated carbon (3) before activation.

7. The disposable warmer of claim 6, **characterized in that**
the low active substance is a carbonaceous material.

8. The disposable warmer of any one of claims 1-7, **characterized in that**
the activated carbon (3) is an oxidation reaction acceleration agent.

## Patentansprüche

1. Einwegwärmer, umfassend:
einen luftdurchlässigen Beutel (1),
eine exotherme Zusammensetzung, die ein Eisenpulver, einen aktivierten Kohlenstoff (3), ein Salz, ein wasserabsorbierendes Harz und Wasser beinhaltet,
einen Duftstoffmaterial (4) und
einen Duftstoffmaterial-Adsorptionshemmstoff (5) zum Hemmen der Adsorption des Duftstoffmaterials (4) in den aktivierten Kohlenstoff (3),
wobei die exotherme Zusammensetzung, das Duftstoffmaterial (4) und der Duftstoffmaterial-Adsorptionshemmstoff (5) in dem luftdurchlässigen Beutel (1) eingeschlossen sind, und
wobei der Durchschnittswert der Jodadsorptionsleistung des aktivierten Kohlenstoffs (3) und der Jodadsorptionsleistung des Duftstoffmaterial-Adsorptionshemmstoffs (5) auf 510-700 mg/g eingestellt ist,
**dadurch gekennzeichnet, dass**
das Mischungsverhältnis des aktivierten Kohlenstoffs (3) und des Duftstoffmaterial-Adsorptionshemmstoffs (5) auf 1,4:1 bis 4:1 eingestellt ist.

2. Einwegwärmer nach Anspruch 1, **dadurch gekennzeichnet, dass**
ein Duftstoffmaterial (4), welches dasselbe wie das Duftstoffmaterial (4) ist oder sich davon unterscheidet, an dem luftdurchlässigen Beutel (1) angebracht oder in diesem eingeschlossen ist.

3. Einwegwärmer, umfassend:
einen luftdurchlässigen Beutel (1), an dem ein Duftstoffmaterial (4) angebracht ist,
eine exotherme Zusammensetzung, die ein Eisenpulver, einen aktivierten Kohlenstoff (3), ein Salz, ein wasserabsorbierendes Harz und Wasser beinhaltet, und
einen Duftstoffmaterial-Adsorptionshemmstoff (5) zum Hemmen der Adsorption des Duftstoffmaterials (4) in den aktivierten Kohlenstoff (3),
wobei die exotherme Zusammensetzung und der Duftstoffmaterial-Adsorptionshemmstoff (5) in dem luftdurchlässigen Beutel (1) eingeschlossen sind, an dem das Duftstoffmaterial (4) angebracht ist,
wobei der Durchschnittswert der Jodadsorptionsleistung des aktivierten Kohlenstoffs (3) und der Jodadsorptionsleistung des Duftstoffmaterial-Adsorptionshemmstoffs (5) auf 510-700 mg/g eingestellt ist,
**dadurch gekennzeichnet, dass**
das Mischungsverhältnis des aktivierten Kohlenstoffs (3) und des Duftstoffmaterial-Adsorptionshemmstoffs (5) auf 1,4:1 bis 4:1 eingestellt ist.

4. Einwegwärmer nach Anspruch 3, **dadurch gekennzeichnet, dass**
ein Duftstoffmaterial (4), welches dasselbe wie das Duftstoffmaterial (4) ist oder sich davon unterscheidet, zusätzlich in dem Beutel eingeschlossen ist.

5. Einwegwärmer nach einem der Ansprüche 1 - 4, **dadurch gekennzeichnet, dass**
der Duftstoffmaterial-Adsorptionshemmstoff (5) eine schwach aktive Substanz mit einer Jodadsorptionsleistung beinhaltet, die geringer als die Jodadsorptionsleistung des aktivierten Kohlenstoffs (3) ist.

6. Einwegwärmer nach Anspruch 5, **dadurch gekennzeichnet, dass**
die schwach aktive Substanz ein Präkursor des aktivierten Kohlenstoffs (3) vor der Aktivierung ist.

7. Einwegwärmer nach Anspruch 6, **dadurch gekennzeichnet, dass**
die schwach aktive Substanz ein kohlenstoffhaltiges Material ist.

8. Einwegwärmer nach einem der Ansprüche 1 - 7, **dadurch gekennzeichnet, dass**
der aktivierte Kohlenstoff (3) ein Oxidationsreaktions-Beschleunigungsmittel ist.

## Revendications

1. Réchauffeur jetable comprenant :
un sac perméable à l'air (1) ;
une composition exothermique contenant une poudre de fer, du charbon actif (3), un sel, une résine absorbant l'eau, et de l'eau ;
un matériau de parfum (4) ; et
un agent inhibant l'adsorption du matériau de parfum (5) pour inhiber l'adsorption du matériau de parfum (4) dans le charbon actif (3) ; et
la composition exothermique, le matériau de parfum (4) et l'agent inhibant l'adsorption du matériau de parfum (5) étant enfermés dans le sac perméable à l'air (1),
dans lequel la valeur moyenne de la performance d'adsorption d'iode du charbon actif (3) et de la performance d'adsorption d'iode de l'agent inhibant l'adsorption du matériau de parfum (5) est ajustée à 510-700 mg/g,
**caractérisé en ce que** le rapport de mélange du charbon actif (3) et de l'agent inhibant l'adsorption du matériau de parfum (5) est ajusté à une valeur de 1,4/1 à 4/1.

2. Réchauffeur jetable selon la revendication 1, **caractérisé en ce qu'**un matériau de parfum (4) qui est identique au ou différent du matériau de parfum (4) est attaché à ou inclus dans le sac perméable à l'air (1).

3. Réchauffeur jetable comprenant :
un sac perméable à l'air (1) auquel un matériau de parfum (4) est attaché ;
une composition exothermique contenant une poudre de fer, du charbon actif (3), un sel, une résine absorbant l'eau, et de l'eau ;
un agent inhibant l'adsorption du matériau de parfum (5) pour inhiber l'adsorption du matériau de parfum (4) dans le charbon actif (3) ; et
la composition exothermique et l'agent inhibant l'adsorption du matériau de parfum (5) étant enfermés dans le sac perméable à l'air (1) auquel le matériau de parfum (4) est attaché,
dans lequel la valeur moyenne de la performance d'adsorption d'iode du charbon actif (3) et de la performance d'adsorption d'iode de l'agent inhibant l'adsorption du matériau de parfum (5) est ajustée à 510-700 mg/g,
**caractérisé en ce que** le rapport de mélange du charbon actif (3) et de l'agent inhibant l'adsorption du matériau de parfum (5) est ajusté à une valeur de 1,4/1 à 4/1.

4. Réchauffeur jetable selon la revendication 3, **caractérisé en ce qu'**un matériau de parfum (4) qui est identique au ou différent du matériau de parfum (4) est de plus enfermé dans le sac.

5. Réchauffeur jetable selon l'une quelconque des revendications 1 à 4, **caractérisé en ce que** l'agent inhibant l'adsorption du matériau de parfum (5) comprend une substance faiblement active ayant une performance d'adsorption d'iode inférieure à la performance d'adsorption d'iode du charbon actif (3).

6. Réchauffeur jetable selon la revendication 5, **caractérisé en ce que** la substance faiblement active est un précurseur du charbon actif (3) avant activation.

7. Réchauffeur jetable selon la revendication 6, **caractérisé en ce que** la substance faiblement active est un matériau carboné.

8. Réchauffeur jetable selon l'une quelconque des revendications 1 à 7, **caractérisé en ce que** le charbon actif (3) est un agent accélérant pour une réaction d'oxydation.
